# EUROPÄISCHE PATENTANMELDUNG

(11) **EP 2 116 226 A1**
(43) Veröffentlichungstag der Anmeldung: **11.11.2009**
(21) Anmeldenummer: 09157400.4
(22) Anmeldetag: 06.04.2009
(51) Int. Cl.: A61K 8/81, A61Q 5/02, A61Q 5/06, A61Q 5/12

(54) **Haarpflegeformulierungen mit kationisch amphoteren Polymeren**

(30) Priorität: 08.05.2008 DE 102008001661
(71) Anmelder: Beiersdorf AG, 20253 Hamburg (DE)
(72) Erfinder: Wendicke, Silke, 22303, Hamburg (DE); Heitmann, Birgit, 23881, Breitenfelde (DE); Neuhoff, Henrike, 22359, Hamburg (DE); Wilken, Maren, 22848, Norderstedt (DE); Demitz, Michael, 22529, Hamburg (DE); Koswig, Marie, 21255, Tostedt (DE); Heuer, Björn, 22145, Hamburg (DE)
(74) Vertreter: Porath, Stefan

(57) **Zusammenfassung**

Haarkosmetisches Mittel, enthaltend ein kationisch amphoteres Polymer, enthaltend die Monomere Vinylpyrrolidon, *tert*-Butylacrylat, Methacrylsäure, Dimethylaminopropylmethacrylamid und Methacryloyloxyethyldimethylethyl Ammonium Ethylsulfat, mit den Vorgaben, dass der Anteil Methacrylsäure geringer ist als der Anteil Dimethylaminopropylmethacrylamid und dass das Polymer mit einer geeigneten Säure neutralisiert ist.

## Beschreibung

Shampoos dienen in erster Linie zum Reinigen der Haare, Haarpflegespülungen zur Verbesserung der Kämmbarkeit. Der Verwender stellt aber immer stärkere Ansprüche an zusätzliche Effekte, besonders verstärkt auch an gewisse Styling-Effekte wie z.B. eine Verbesserung des Volumens der Frisur, eine dauerhafte Glättung der Haare oder die Definition von Locken. Solche Styling-Effekte können mit verschiedenen Inhaltsstoffen erreicht werden. Eine Möglichkeit besteht im Einsatz von Styling-Polymeren, die als Lösung oder Suspension eingesetzt werden können. Gelöste Polymere haben den Vorteil der einfachen Formulierbarkeit, verbleiben aber wegen ihrer Löslichkeit nach dem Ausspülen in nur geringer Menge auf dem Haar. Bekannte Conditioner-Polymere lagern sich wegen ihrer positiven Ladung bevorzugt an geschädigten Stellen des Haars an, besitzen aber eine nicht ausreichende Styling-Leistung. Silikone wirken sich positiv auf Kämmbarkeit und Griff aus, liefern aber keinen Styling-Effekt und fühlen sich in höherer Konzentration unangenehm fettig und ölig an. Suspendierte Polymere stellen größere Anfoderungen an die Stabilisierung in der Formulierung, können aufgrund ihrer Wasserfestigkeit aber eher auf dem Haar verbleiben. Generell wirken sich zudem hohe Mengen Polymer auf dem Haar negativ auf die Kämmbarkeit und auf den Griff aus. Ziel der Entwicklung einer Haarpflege-Formulierung mit Styling Effekt ist es daher, einen deutlich merkbaren Styling-Effekt nach Ausspülen der Formulierung zu erhalten, gleichzeitig gute Ergebnisse in den wichtigen Parametern Griff im nassen und trockenen Haar sowie Kämmbarkeit im nassen und trockenen Haar zu erreichen. Dies ist mit bekannten Formulierungen zurzeit nicht möglich.

WO 2008/031892 A1 (BASF) beschreibt die Verwendung von kationisch amphoteren Copolymeren in Haarsprays, auch eine Shampoo-Formel ist angegeben.

US 6297203 B1 (Procter & Gamble) - Kombination von 2 kationischen Polymeren unterschiedlicher Ladungsdichte (0,2 - 2,0 und 2,0 - 4,75 meq/g.

US 6248317 B1 (Procter & Gamble) - wasserunlösliches Styling-Polymer in Kombination mit einem kationischen Deposition Polymer.

US 6180576 B1 - Kombination eines wasserunlöslichen Silikons mit einem amphoteren Conditioner-Polymer.

EP 1115370 B1 (Calgon) - Kombination eines wasserunlöslichen Silikons mit einem amphoteren Polymer.

EP 1366741 B1 (L'Oreal) - Kombintaion eines kationischen oder amphoteren Polymers mit einem speziellen Block-Copolymer.

US 6524614 B2 (L'Oreal) - Kombination eines speziellen Tensidsystems mit Polymeren für einen Styling-Benefit aus Shampoo Formulierungen.

Es hat sich nun gezeigt, dass die Aufgabe gelöst wird durch ein haarkosmetisches Mittel, enthaltend ein kationisch amphoteres Polymer, enthaltend die Monomere Vinylpyrrolidon, *tert-*Butylacrylat, Methacrylsäure, Dimethylaminopropylmethacrylamid und Methacryloyloxyethyldimethylethyl Ammonium Ethylsulfat, mit den Vorgaben, dass der Anteil Methacrylsäure geringer ist als der Anteil Dimethylaminopropylmethacrylamid und dass das Polymer mit einer geeigneten Säure neutralisiert ist und mindestens ein weiterer Stoff der den Zustand des Haares nach Applikation verbessert. Die Formulierungen liefern einen angenehmen Griff, gute Kämmbarkeit im nassen und trockenen Haar, sowie einen ausgeprägten Styling-Effekt. Besonders bevorzugt ist es, wenn das kationisch amphoteres Polymer zusammengesetzt aus 45 bis 49 Gew.-% *tert*-Butylacrylat, 23 bis 27 Gew.-% Vinylpyrrolidon, 9 bis 11 Gew.-% Dimethylaminopropylmethacrylamid, 13 bis 17 Gew.-% Methacryloyloxyethyldimethylethyl Ammonium Ethylsulfat, 2 bis 4 Gew.-% Methacrylsäure. Ganz besonders bevorzugt ist es, wenn das kationisch amphotere Polymer aus 47 Gew.-% tert. Butylacrylat, 25 Gew.-% Vinylpyrrolidon, 10 Gew.-% Dimethylaminopropylmethacrylamid, 15 Gew.-% Methacryloyloxyethyldimethylethyl Ammonium Ethylsulfat, 3 Gew.-% Methacrylsäure zusammengesetzt ist. Dabei ist besonders bevorzugt, wenn das kationisch amphotere Polymer ein Molekulargewicht von 80 000 bis 240 000 g/mol aufweist. Weiter ist es besonders bevorzugt, wenn das haarkosmetisches Mittel zusätzlich 2 - 25% eines Tensids, ausgewählt unter den nichtionischen, anionischen, kationischen oder amphoteren Tensiden enthält. Die Erfindung umfasst auch die Verwendung eines haarkosmetischen Mittels wie oben beschrieben zum Erreichen eines Styling-Effekts, und zwar zur Verbesserung des Frisurvolumens nach Ausspülen des haarkosmetischen Mittels. Dieser Styling-Effekt besteht insbesondere in einer überraschenden Verbesserung des Volumens der Frisur und wurde von geschulten Panelisten im Halbseitenvergleich gegenüber Formulierungen ohne das Polymer evaluiert.

Haarkosmetische Mittel zur Reinigung des Haares werden auch als Schampoos bezeichnet. Bei diesen ist es bevorzugt, wenn sie anionische Teside enthalten, besonders bevorzugt in Gehalten von 4 bis 15 Gew.-%. Bevorzugt enthalten solche haarkosmetischen Mittel zur Reinigung des Haares zusätzlich amphotere Tenside besonders bevorzugt Acyl-/dialkylethylendiamine, insbesondere Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat. Bevorzugt enthalten solche haarkosmetisches Mittel zur Reinigung des Haares als anionische Teside Alkylethersulfate, insbesondere Laurylethersulfat in Kombination mit Cocamidopropylbetain, besonders bevorzugt die Kombination Alkylethersulfate, Cocamidopropylbetain und Alkylpolyglykolestersulfosuccinate, ganz besonders bevorzugt Laurylethersulfat, Cocamidopropylbetain und Alkylpolyglykolestersulfosuccinate.

Haarkosmetisches Mittel zur Pflege des Haares werden als Conditioner bezeichnet. Diese sind keine Schampoos und enthalten kationische Teside, bevorzugt zu einem Gehalt von 0,1 bis 2 Gew.-%. Weiter ist bevorzugt, wenn sie zusätzlich Filmbildner enthalten. Weiter ist bevorzugt, wenn sie als kationische Teside Alkylamine, Alkylimidazole, Ethoxylierte Amine, Quaternäre Tenside, Esterquats enthalten. Weiter ist bevorzugt, wenn sie als Filmbildner Cellulosederivate, quaternisierte Guar Gum Derivate und Dimethyldiallylammoniumchlorid-Acrylamid Copolymere enthalten.

Als Tenside können wie gesagt anionische, kationische, amphotere und nichtionische Tenside eingesetzt werden in Gesamtkonzentrationen von 2 bis 25 Gew.-%.

Besonders vorteilhaft zur Herstellung von Schampoos ist eine Kombination von Alkylethersulfaten (besonders Laurylethersulfat) und Cocamidopropylbetain, besonders bevorzugt Alkylethersulfate, Cocamidopropylbetain und Alkylpolyglykolestersulfosuccinate, ganz besonders bevorzugt Laurylethersulfat, Cocamidopropylbetain und Alkylpolyglykolestersulfosuccinateeinzusetzen. Besonders vorteilhaft ist Laurylethersulfat in Konzentrationen von 5 - 15 Gew.-% und Cocamidopropylbetain in Konzentration von 0.5 - 10 Gew.-% einzusetzen. Ganz besonders vorteilhaft ist es Laurylethersulfat in Konzentrationen von 6 - 12 Gew.-% und Cocamidopropylbetain in Konzentrationen von 1 - 5 Gew. % einzusetzen.

Als ebenso sehr vorteilhaft für Schampoos hat sich die Kombination von Laurylethersulfat mit Cocamidopropylbetain und Decyl Glucosid erwiesen mit Konzentrationen von 2 - 12 Gew.-% Laurylethersulfat, 2 - 10 Gew.-% Cocamidopropylbetain und 2 - 5 Gew.-% Decyl Glucosid. Ebenfalls vorteilhaft für Schampoos zu verwendende amphotere Tenside sind: Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat.

Anionische Tenside im Sinne der Erfindung weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wässrigem Medium keine Ionen.

### A. Anionische Tenside

Erfindungsgemäß vorteilhaft zu verwendende anionische Tenside sind Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-Iauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z. B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido-MEA-Sulfosuccinat
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
2. Alkylsulfate, beispielsweise Natrium-, Ammonium- und TEA-Laurylsulfate

### B. Amphotere Tenside

1. Erfindungsgemäß vorteilhaft zu verwendende amphotere Tenside sind
2. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
3. N-Alkylaminosäuren, beispielsweise Aminopropylalkylglutamid, Alkylaminopropionsäure, Natriumalkylimidodipropionat und Lauroamphocarboxyglycinat.

Vorteilhafte nichtionische Tenside mit einem HLB-Wert von 10 bis 20 können beispielsweise sein:
PEG-20 Mandelölglyzerid, PEG-20 Nachtkerzenölglyzerid, PEG-60 hydrogeniertes Rizinusöl, Polysorbat 60, PEG-20 Sorbitan Isostearat, PEG-40 hydrogeniertes Rizinusöl, Polysorbat 80, Succrose Cocoat, PEG-45 Palmkernölglyzeride, PEG-45 Distelölglyzerid, PEG-60 Nachtkerzenölglyzerid, PEG-42 Babassuölglyzerid, PEG-20 Rizinusoleat, Steareth-21, Oleth-20, Polysorbat 40, Laureth-9, Laureth-15, Ceteareth-20, Ceteth-20, PEG-12 Laurat, PEG-24 Stearat, PEG-20 Stearat, PEG-20 Oleat, PEG-75 Lanolin, Laneth-40, PEG-8 Dilaurat, Polysorbat 65, PEG-7 Glyceryl Cocoat, Laneth-10, PEG-12 Oleat, Polyglyzeryl-6 Laurat, Polyglyzeryl-10 Laurat, Polyglyzeryl-10 Myristat, Polyglyzeryl-10 Stearat, Polyglyzeryl-10 Oleat, Polyglyzeryl-10 Isostearat, Polyglyzeryl-10 Diisostearat, PEG-15 Glyzeryloleat, PEG-60 Sorbitan Tetraoleat, PEG-10 Oleylether

Neben Parfüm- und Farbstoffen sowie einer Reihe von Verbindungen, welche die Haltbarkeit der Zubereitungen erhöhen, werden in jüngerer Zeit den Haarshampoos unterschiedliche Arten von Wirkstoffen zugefügt. Hierzu zählen neben UV-Absorbern, Vitaminen oder Pflanzenextrakten auch sogenannte Haarkonditionierer (engl. conditioner), welche das Haar pflegen und seine Kämmbarkeit und seinen Griff verbessern sowie seinen Glanz erhöhen. Konditionierer ziehen, im Gegensatz zu den meisten anderen Bestandteilen von Shampoos, auf das Haar auf und verbleiben dort nach dem Spülen (Filmbildner). Sie lagern sich aufgrund ihres Molekülaufbaus an die Schadstellen der Cuticula des Haares und glätten das Haar. Dadurch wird das Haar weniger rauh und spröde, die Frisur bekommt deutlich mehr Glanz und lässt sich leichter kämmen. Auch wird das Haar weniger empfindlich für eine elektrostatische Aufladung. Die wichtigsten haarkonditionierenden Substanzen in Shampoos stellen die polymeren quatären Ammoniumverbindungen dar. Auch können kationische Cellulosederivate und Polysaccharide eingesetzt werden. Weiterhin werden auch Silikonverbindungen zur Konditionierung eingesetzt.

Erfindungsgemäß vorteilhafte Filmbildner stellen Cellulosederivate, quaternisierte Guar Gum Derivate und Dimethyldiallylammoniumchlorid-Acrylamid Copolymere dar.

Bei den kationischen Hydroxyethylcellulosederivaten haben sich Polyquaternium 10 Typen mit einem Molekulargewicht von 400.000 g/mol und einer mittleren Ladungsdichte von 1,07 - 1,57 als geeignet gezeigt. Als geeignet haben sich auch Polyquarternium-10 mit einem Molekulargewicht von 250.000 g/mol und einer höheren Ladungsdichte von 1,7meg/g gezeigt.

Bei den quaternisierte Guar Gum Derivate haben sich insbesondere Guar Hydroxypropyltrimoniumchlorid (z.B. Jaguar Excel®, Jaguar C 162® der Firma Rhodia, CAS 65497-29-2, CAS 39421-75-5) dar. Das Molekulargewicht dieser Derivate liegt typischer Weise in einem Bereich von 50.000 bis 2.500.000. Als besonders geeignet haben sich Molekulargewichte von 1.000.000 bis 1.500.000 gezeigt. Insbesonders geeinet ist Guar Hydroxypropyltrimonium Chlorid mit einem Molekulargewicht von 1.300000 und einer Ladungsdichte von 0.4-0.6 meq/g.

Als Konditioniermittel besonders geeignet haben sich Dimethyldiallylammoniumchlorid-Acrylamid Copolymere mit einem Molekulargewicht von 1,4x10⁶-1,8x10⁶ g/mol und einer Ladungsdichte von 2,5 - 3,5 gezeigt.

Als sehr gut hat sich auch die Kombination von Dimethyldiallylammoniumchlorid-Acrylamid Copolymere mit kationischen Hydroxyethylcellulosederivaten gezeigt. Bei der Kombination von Dimethyldiallylammoniumchlorid-Acrylamid Copolymer und Polyquaternium-10 haben sich Einsatzkonzentrationen von jeweils 0,01 - 1 % als vorteilhalft gezeigt. Besonders geeignet waren Einsatzkonzentrationen von 0,01 - 0,5 %.

Bei der Kombination von Dimethyldiallylammoniumchlorid-Acrylamid Copolymer und Guar Hydroxypropyltrimonium Chlorid haben sich Einsatzkonzentrationen von jeweils 0,01 - 1% als vorteilhalft gezeigt. Besonders geeignet waren Einsatzkonzentrationen von 0,01 - 0,5 %.

Als Verdicker werden bevorzugt Natriumchlorid, PEG-200 hydrogeniertes Glycerylpalmitat und Acrylat Copolymere verwendet. Besonders vorteilhaft sind Einsatzkonzentrationen von 0.05 - 5 Gew.-%.

Als Perlglanzsysteme können prinzipiell alle gängigen, bekannten Perlglanzrohstoffe verwendet werden. Besonders vorteilhaft ist die Verwendung von PEG-3 Distearat. Ganz besonders vorteilhaft ist es, wenn dieser Perlglanzrohstoff eine Teilchengröße aufweist in der Art, dass 90 % der Volumenanteile eine Teilchengröße von kleiner 50µm aufweisen. Die Einsatzkonzentrationen des Perlglanzes liegen vorteilhafterweise bei 0,5 - 15 Gew.-%.

Anstelle des Perlglanzsystems kann auch ein Trübungsmittel eingesetzt werden. Besonders vorteilhaft ist der Einsatz von Mischungen, bestehend aus Glycol Distearat, Coco-Glucosid, Glyceryl Oleat, Glyceryl Stearate wie sie unter dem Handelsnamen Lamesoft TM Benz (Cognis) angeboten werden.

Vorteilhaft kann auch eine Kombination von Perlglanz und Trübungsmittel eingesetzt werden.

Die erfindungsgemäßen waschaktiven Zubereitungen zeichnen sich in der Regel durch einen Wassergehalt von 95 - 5 Gew.-% aus, bezogen auf das Gesamtgewicht der Zubereitungen und stellen Gele dar.

Zubereitungen gemäß der Erfindung sind vorteilhaft auf einen pH-Bereich > 4,2 gepuffert, besonders bevorzugt > 4,5 besonders bevorzugt 4,8 - 7,5.

Die Herstellung der erfindungsgemäßen kosmetischen und/oder dermatologischen Zubereitungen erfolgt in der dem Fachmann üblichen Weise, zumeist dergestalt, dass die erfindungsgemäß verwendeten grenzflächenaktiven Stoffe bei gleichmäßigem Rühren und gegebenenfalls unter Erwärmen suspendiert und gewünschtenfalls homogenisiert, gegebenenfalls mit weiteren Lipidkomponenten und gegebenenfalls mit einem oder mehreren weiteren Emulgatoren vereinigt, hernach die Ölphase mit der wässrigen Phase, in welche gegebenenfalls ein Verdickungsmittel eingearbeitet worden ist, und welche vorzugsweise etwa die gleiche Temperatur besitzt wie die Ölphase, vermischt, gewünschtenfalls homogenisiert und auf Raumtemperatur abkühlen lässt. Nach Abkühlen auf Raumtemperatur kann, insbesondere, wenn noch flüchtige Bestandteile eingearbeitet werden sollen, nochmaliges Homogenisieren erfolgen.

Im Fall von Haarspülungen und Haarkuren werden als Tenside quaternäre Tenside wie zum Beispiel Alkylamine, Alkylimidazole,Ethoxylierte Amine, Quaternäre Tenside, Esterquats eingesetzt.

Quaternäre Tenside erhalten mindestens ein N-Atom, das mit 4 Alkyl- oder Arylgruppen kovalent verbunden ist. Dies führt, unabhängig vom pH-Wert, zu einer positiven Ladung. Die erfindungsgemäß verwendeten kationischen Tenside können ferner bevorzugt gewählt werden aus der Gruppe der quaternären Ammoniumverbindungen, insbesondere Benzyltrialkylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylamidopropyltrimethylammoniumchlorid, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Monomere Ammonium-Verbindungen werden vielfach in Haarspülungen und Haarkuren z. B. in Konzentrationen von 0,5 - 5 Gew.-% eingesetzt. In leave on Produkte werden monomere oder polymere quartäre Ammonium-Verbindungen z.B. in Konzentrationen von 0,1 - 0,5 Gew.-% eingesetzt. Dazu gehören zum Beispiel neben anderen Ammonium-Verbindungen Cetrimonium Chloride wie es unter der Bezeichnung Dehyquart A von der Fa. Henkel angeboten wird oder Distearoylethyl Hydroxyethylmonium Methosulfate wie es unter der Bezeichnung Dehyquart F 75 von der Fa. Henkel angeboten wird.

Im Fall von Haarspitzenfluiden kann auf den Tensidgehalt sogar verzichtet werden und statt der Tenside Silikonöle wie zum Beispiel Cyclomethicon und/oder Dimethiconol eingesetzt werden.

Es ist von Vorteil, wenn zusätzlich weitere Styling-Polymere eingesetzt werden, ausgewählt aus der Gruppe der nichtionischen, kationischen, anionischen oder amphoteren Polymere, wie z. B. Polymere mit der INCI Acrylates Copolymer (Luvimer®, Luviflex® Soft von BASF, Balance CR®, Balance O/55® von National Starch, Avalure AC® von Lubrizol), Acrylates/Hydroxyesters Acrylates Copolymer (Acudyne® von Rohm & Haas), Acrylates/Octylacrylamide Copolymer (Amphomer® oder Resyn XP von National Starch), AMP-Acrylates/Allyl Methacrylates Copolymer (Fixate® von Lubrizol), Butyl Ester of PVM/MA Copolymer (Gantrez® von ISP), Ethyl Ester of PVM/MA Copoylmer (Gantrez®, Omnirez® oder Advantage® von ISP), Acrylamide/Sodium Acryloyldimethyltaurate/Acrylic Acid Polymer (Acudyne SCP® von Rohm & Haas), Isobutylene/Ethylmaleimide/Hydroxyethylmaleimide Copolymer (Aquaflex FX-64® von ISP), Octylacrylamide/Acrylates/Butylaminoethyl Methacrylate Copolymer (Amphomer® oder Balance® von Natinal Starch), Acrylates/t-Butylacrylamid Copolymer (Ultrahold® von BASF), PEG/PPG-25/25 Dimethicone/Acrylates/t-Butylacrylates Copolymer (Luviflex Silk® von BASF), Polyvinylcaprolactam (Luviskol Plus® von BASF), PVM/MA Copolymer (Luviform FA® von BASF), PVP (Luviskol® von BASF), PVP/VA Copolymer (Luviskol® von BASF), VP/Dimethylaminoethylmethacrylate Copolymer (Copolymer® von ISP), Sodium Polystyrene Sulfonate (Flexan® von National Starch), VA/Crotonates/Vinyl Neodecanoate Copoylmer (Luviset CAN® von BASF, Resyn® von Natinal Starch), VA/Crotonates/Vinyl Propionate Copolymer (Luviset CAP® von BASF), VA/Butyl Maleate/Isobornyl Acrylate Copolymer (Advantage Plus® von ISP), VA/Crotonates Copolymer (Luviset CA 66® von BASF, Aristoflex® von Clariant), VA/Vinylbutylbenzoate/Crotonates Copolymer (Mexomere PW® von Chimex), Vinylcaprolactam/PVP/Dimethylaminoethyl Methacrylate Copolymer (Advantage® von ISP), VA/Butyl Maleate/Isobornyl Acrylate Copolymer (Advantage Plus® von ISP), Acrylates/C1-2 Succinates/HydroxyAcrylates Copolymer (Allianz LT-120® von ISP), VP/Vinyl Caprolactam/DMAPA Acrylates Copolymer (Aquaflex SF-40® von ISP), Vinyl Caprolactam/VP/Dimethylaminoethyl Methacrylate Copolymer (Gaffix® von ISP), VP/DMAPA Acrylates Copolymer (Styleze CC-10 von ISP), VP/Acrylates/Lauryl Methacrylate Copolymer (Styleze 2000® von ISP), VP/Methacrylamide/N-Vinyl Imidazole Copolymer (Luviset Clear® von BASF), alle Polyacrylat-Typen (Fixate Plus® und Fixate Superhold von Lubrizol, Luviset Shape® von BASF), alle Polyquaternium-Typen (Celquat® von National Starch, Gafquat® von ISP, Luviquat® von BASF, Styleze W-20® von ISP, Aquastyle 300® von ISP), und viele mehr. Auch andere Polymerklassen, wie Polyurethane, z.B. Polyurethane-1 (Luviset PUR® von BASF), Polyurethane-2 (Avalure UR® von Lubrizol), Polyurethane-6 (Luviset SI PUR® von BASF), Polyester, z.B. Polyester-5 (AQ-Polymere von Eastman), Polyimide, z.B. Polyimid-1 (Aquaflex XL-30® von ISP), und viele mehr, können verwendet werden.

Ein weiterer Aspekt der Erfindung ist die Verwendung dieses Polymers in Shampoos zur Erzeugung von Volumen.

Ein weiterer Aspekt der Erfindung ist die Verwendung dieses Polymers in Shampoos zur Definition von Locken.

Ein weiterer Aspekt der Erfindung ist die Verwendung dieses Polymers in Shampoos zum Erreichen eines Straightening-Effekts.

Ein weiterer Aspekt der Erfindung ist die Verwendung dieses Polymers in Shampoos zum Erreichen von Haarverdickung bzw. Haarstärkung.

Ein weitere Aspekt der Erfindung ist die Verwendung dieses Polymers in Shampoos zum Erreichen einer besseren Frisierbarkeit.

Ein weiterer Aspekt der Erfindung ist die Verwendung dieses Polymers in Spülungen oder Kuren zur Erzeugung von Volumen.

Ein weiterer Aspekt der Erfindung ist die Verwendung dieses Polymers in Spülungen oder Kuren zur Definition von Locken.

Ein weiterer Aspekt der Erfindung ist die Verwendung dieses Polymers in Spülungen oder Kuren zum Erreichen eines Straightening-Effekts.

Ein weiterer Aspekt der Erfindung ist die Verwendung dieses Polymers in Spülungen oder Kuren zum Erreichen von Haarverdickung bzw. Haarstärkung.

Ein weitere Aspekt der Erfindung ist die Verwendung dieses Polymers in Spülungen oder Kuren zum Erreichen einer besseren Frisierbarkeit.

Shampoos sind in diesem Zusammenhang Zusammensetzungen, die reinigende Tenside enthalten. Dieses Zusammensetzungen können auch als 2 in 1 Produkte mit zusätzlichem Haar-Conditioner oder auch als 2 in 1 Produkte zur Reinigung von Haar und Körper vorliegen. Es ist im Sinne der Erfindung unerheblich, ob es sich um ein klassisches Shampoo, ein Konzentrat oder Trockenshampoo handelt.

Spülungen und Kuren sind in diesem Zusammenhang Zusammensetzungen zur Anwendung auf dem Haar, deren Hauptaufgabe nicht die Reinigung, sondern die Verbesserung von Kämmbarkeit, Griff oder anderen sensorischen des Haares ist und die nach einer Shampoo-Behandlung im Haar angewendet werden. Diese Zusammensetzungen können alle gängigen Conditioner-Stoffe, wie z. B. kationische Tenside, kationische Polymer, Silikone, Silikon-Copolymere usw. enthalten. Es ist im Sinne der Erfindung unerheblich, ob diese Produkte als Lösung, Gel, Balm, Lotion, Creme, Fluid, Spray, Aerosol, Aerosol-Schaum, Hydrodispersion, W/O-Emulsion, O/W-Emulsion, W/Si-Emulsion, Si/W-Emulsion oder sonstige Emulsion vorliegt. Es ist unerheblich, ob diese Zusammensetzungen nach der Anwendung ausgespült werden oder im Haar verbleiben.

### Beispiele

**Polymer 1:** statistisches, lineares Copolymer aus Vinylpyrrolidon, *tert*-Butylacrylat, Methacrylsäure, Dimethylaminopropylmethacrylamid und Methacryloyloxyethyldimethylethyl Ammonium Ethylsulfat, neutralisiert mit Phosphorsäure auf pH 6,0. Das Polymer besteht aus 25 Gew.-% Vinylpyrrolidon, 47 Gew.-% *tert*-Butylacrylat, 3 Gew.-% Methacrylsäure, 10 Gew.-% Dimethylaminopropylmethacrylamid und 15 Gew.-% Methacryloyloxyethyldimethylethyl Ammonium Ethylsulaft.

Die Mengenangaben in den Beispielen sind Gewichts-Prozent.

**Perlglänzendes Shampoo**

| | **1** | **2** | **3** | **4** | **5** | **6** | **7** | **8** |
|---|---|---|---|---|---|---|---|---|
| Natrium Laurylethersulfat | 9 | 5 | 7 | 9 | 9 | 10 | 12 | 15 |
| Cocamidopropyl Betain | 2 | 5 | 4 | 3,5 | 5 | 3 | 3 | 5 |
| Di-Natrium PEG-5 Laurylcitrat Sulfosuccinat | 3,5 | - | 4 | 3 | 3 | 2 | - | 4 |
| Decyl Glucoside | - | 3 | - | - | - | - | 3 | - |
| Polyquaternium-10 | - | 0,3 | 0,2 | 0,3 | 0,1 | 0,1 | 0,4 | 0,2 |
| Polyquaternium-7 | 0,3 | - | 0,3 | - | - | 0,2 | - | 0,5 |
| PEG-200 Hydrogenated Glyceryl Palmate | - | 0,8 | 1 | - | 1 | 0,5 | - | 1,5 |
| Guar Hydroxypropyltrimoniumchlorid | 0,1 | 0,1 | - | 0,1 | 0,1 | - | - | - |
| PEG-90M | 0,3 | 0,05 | 0,2 | 0,1 | 0,05 | 0,1 | 0,2 | 0,02 |
| PEG-40 hydrogeniertes Rizinusöl | 0,5 | 0,8 | 0,6 | 0,6 | 0,9 | 0,6 | 0,5 | 0,7 |
| PEG-3 Distearat | - | 2 | - | 1,5 | 1 | - | - | 3 |
| Glycol Distearat | 3 | - | 1,5 | - | - | 2 | 1 | - |
| PEG-7 Glycerylcocoat | 1,5 | - | - | - | - | - | - | 2 |
| Trisodium EDTA | - | 0,5 | - | - | 0,7 | - | - | - |
| **Polymer 1** | 0.5 | 1.0 | 0.7 | 0.3 | 0.1 | 0.6 | 0.8 | 0.2 |
| Tetrasodium Iminodisuccinat | - | - | 0,7 | - | - | 0,5 | - | - |
| Natronlauge | q.s. | q.s. | q.s. | q.s. | q.s. | q.s. | q.s. | q.s. |
| Natrium Salicylat | 0,2 | 0,4 | 0,2 | 0,2 | 0,4 | 0,2 | 0,2 | 0,4 |
| Natrium Benzoat | 0,45 | 0,4 | 0,4 | 0,4 | 0,4 | 0,4 | 0,45 | 0,4 |
| Parfüm | q.s. | q.s. | q.s. | q.s. | q.s. | q.s. | q.s. | q.s. |
| Benzophenon-4 | - | - | - | - | 0,25 | - | 0,1 | - |
| Oryzanol | - | - | - | 0,1 | - | 0,05 | - | - |
| Lotusblütenextrakt | 0,1 | - | - | - | - | - | 0,05 | - |
| Citronensäure | q.s. | q.s. | q.s. | q.s. | q.s. | q.s. | q.s. | q.s. |
| Natriumchlorid | q.s. | q.s. | q.s. | q.s. | q.s. | q.s. | q.s. | q.s. |
| Avocodoöl | 0,3 | - | - | - | - | - | - | 0,5 |
| Wasser | ad 100 | ad 100 | ad 100 | ad 100 | ad 100 | ad 100 | ad 100 | ad 100 |

**Klares Shampoo**

| | **9** | **10** | **11** | **12** | **13** | **14** | **15** |
|---|---|---|---|---|---|---|---|
| Natrium Laurylethersulfat | 10 | 7 | 5 | 9 | 12 | 15 | 9 |
| Di-Natrium PEG-5 Laurylcitrat Sulfosuccinat | 3 | - | 4 | 3 | 3 | 2 | - |
| Decyl Glucoside | - | 2 | 3 | - | - | - | 3 |
| Polyquaternium-10 | - | 0,2 | - | 0,3 | - | 0,5 | 0,2 |
| Polyquaternium-7 | 0,3 | - | 0,3 | - | - | 0,2 | - |
| PEG-200 Hydrogenated Glyceryl Palmate | 1 | 0,5 | 1,5 | 1,5 | 0,5 | 1 | 1,5 |
| Guar Hydroxypropyltrimoniumchlorid | 0,1 | - | 0,2 | 0,1 | 0,3 | - | - |
| PEG-90M | 0,1 | 0,2 | 0,05 | 0,1 | 0,2 | 0,05 | 0,3 |
| PEG-40 hydrogeniertes Rizinusöl | 0,5 | 0,7 | 0,9 | 0,5 | 0,6 | 0,8 | 0,5 |
| PEG-7 Glycerylcocoat | 2 | - | - | - | - | 2 | - |
| Trisodium EDTA | 0,7 | - | - | - | - | - | 0,5 |
| Tetrasodium Iminodisuccinat | - | - | 0,5 | - | - | 0,7 | - |
| **Polymer 1** | 0,5 | 1.0 | 0,5 | 0,5 | 1,0 | 0,5 | 0,5 |
| Natronlauge | q.s. | q.s. | q.s. | q.s. | q.s. | q.s. | q.s. |
| Natrium Salicylat | - | - | 0,2 | - | - | - | 0,2 |
| Natrium Benzoat | 0,4 | 0,45 | 0,4 | 0,4 | 0,4 | 0,45 | 0,4 |
| Parfüm | q.s. | q.s. | q.s. | q.s. | q.s. | q.s. | q.s. |
| Benzophenon-4 | - | 0,25 | - | - | 0,1 | - | - |
| Oryzanol | 0,05 | - | - | 0,1 | - | - | - |
| Kamillenextrakt | - | - | 0,05 | - | - | - | 0,03 |
| Citronensäure | q.s. | q.s. | q.s. | q.s. | q.s. | q.s. | q.s. |
| Avocodoöl | 1 | - | - | - | - | 0.5 | - |
| Natriumchlorid | q.s. | q.s. | q.s. | q.s. | q.s. | q.s. | q.s. |
| Wasser | ad 100 | ad 100 | ad 100 | ad 100 | ad 100 | ad 100 | ad 100 |

**Mildes Shampoo**

| | **16** | **17** | **18** | **19** | **20** | **21** |
|---|---|---|---|---|---|---|
| Natrium Myristylethersulfat | 4 | 4 | 5 | 5 | 4 | 4 |
| Decylglucosid | 4 | 4 | 4 | 4 | 4 | 2 |
| Dinatrium PEG-5 Laurylcitrat Sulfosuccinat | 4 | 4 | 3 | 5 | 5 | 3 |
| PEG-80 Sorbitan Laurat | 2 | 1 | 1 | - | 0.5 | - |
| PEG-200 Hydrogenated Glyceryl Palmate | - | - | - | 0.2 | 0.3 | 0,4 |
| Polyquaternium-7 | 0.3 | 0.1 | 0.2 | 0.3 | 0,4 | 0,5 |
| Polyquaternium-10 | 0,2 | 0.3 | 0,1 | 0,2 | 0.2 | 0,1 |
| PEG-3 Distearat | 1 | 0,75 | 0,5 | 1,5 | - | - |
| Glycol Distearat | - | - | - | - | 0.5 | 0,5 |
| PEG-90M | 0,05 | 0,75 | 0,1 | 0,05 | 0,1 | 0,05 |
| **Polymer 1** | 0,5 | 0,5 | 0,5 | 0,5 | 0,5 | 0,5 |
| PEG-40 hydriertes Rizinusöl | 0.2 | 0.3 | 0.4 | 0.2 | 0.3 | 0,4 |
| Natrium Salicylat | 0.2 | 0.2 | 0.2 | 0.4 | 0.4 | 0,4 |
| Natrium Benzoat | 0.4 | 0.4 | 0.4 | 0.4 | 0.4 | 0,4 |
| Natriumchlorid | 0.9 | 1.0 | 1.2 | 1,5 | 1,8 | - |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

**Spülung**

| | **22** | **23** | **24** | **25** | **26** | **27** |
|---|---|---|---|---|---|---|
| Methylparaben | 0,25 | 0,25 | 0,25 | 0,25 | 0,25 | 0,25 |
| Propylparaben | 0,1 | 0,1 | 0,1 | 0,1 | 0,1 | 0,1 |
| Aqua | ad 100 | ad 100 | ad 100 | ad 100 | ad 100 | ad 100 |
| Cetearyl Alcohol | | 3,5 | | | 5,24 | |
| Cetyl Alcohol | 2,5 | | 1,4 | 2 | | 2,5 |
| Lactic Acid | 0,8 | | 0,7 | 0,8 | | 0,78 |
| Benzophenone-4 | | | | | 0,25 | |
| Glycerin | | 5 | | | 5 | |
| Aqua + Sodium Hydroxide | | | | | 0,15 | |
| **Polymer 1** | 1,0 | 1,0 | 1,0 | 1,0 | 1,0 | 1,0 |
| Sodium Chloride | 0,01 | 0,01 | 0,01 | 0,01 | | 0,01 |
| Stearyl Alcohol | 4,8 | | 2,8 | 3,8 | | 4,8 |
| Distearoylethyl Hydroxyethylmonium Methosulfate | | 2,86 | | | 2,86 | |
| Oryzanol | 0,05 | 0,05 | | 0,05 | 0,05 | 0,05 |
| PEG-90 M | | 0,05 | | 0,05 | | |
| Palmitamidopropyltrimonium Chloride | | 1,66 | | 0,415 | 1,66 | |
| Coco Betaine | 0,85 | 0,85 | | | | 0,85 |
| PPG-3 Benzyl Ether Myristate | | | 1,5 | 2 | 2 | |
| Stearamidopropyl Dimethylamine | 2,2 | | 2,2 | 2 | | 2,2 |
| Parfum | 0,7 | 0,7 | 0,7 | 0,7 | 0,7 | 0,7 |
| Dimethicone | 4,6 | | | | | 4,4 |
| Propellant | | 4 | 9 | | | |

**Balm**

| | **28** | **29** | **30** | **31** | **32** |
|---|---|---|---|---|---|
| Aqua | ad 100 | ad 100 | ad 100 | ad 100 | ad 100 |
| Citric Acid | 0,5 | 0,01 | 0,01 | 0,01 | 0,5 |
| Sodium Benzoate | 0,4 | 0,2 | 0,2 | 0,2 | 0,4 |
| PEG-12 | | 4,5 | 4,5 | 4,5 | |
| Panthenol | 0,1 | 0,2 | 0,2 | 0,2 | 0,1 |
| Benzophenone-4 | 0,03 | 0,05 | 0,05 | 0,05 | 0,03 |
| Glycerin | | 0,869 | | 0,869 | |
| PEG-40 Hydrogenated Castor Oil | 0,3 | 0,3 | 0,3 | 0,3 | 0,3 |
| Hydroxyethylcellulose | | 0,8 | 0,8 | 0,8 | |
| Alcohol Denat. | 12 | 10 | | | 10 |
| PEG-12 Dimethicone | 1 | | 1 | 1 | 1 |
| Chitosan Lactate | | 5 | 10 | | 10 |
| Cetrimonium Chloride | | 0,3 | 0,3 | 0,3 | |
| Polyquaternium-4 | | 2 | | 1 | |
| VP/VA Copolymer | | | | 2 | |
| Hydroxypropyl Guar | 1,3 | | | | 1,3 |
| Sodium Salicylate | 0,2 | | | | 0,2 |
| PEG/PPG-17/18 Dimethicone | | 0,1 | 0,1 | | |
| **Polymer 1** | 1,0 | 1,0 | 1,5 | 1,0 | 1,0 |
| VP/Methacrylamide/Vinyl Imidazole Copolymer | 12,5 | | | | 12,5 |
| Polyquaternium-68 | | | | 10 | |
| Parfum | 0,3 | 0,2 | 0,2 | 0,2 | 0,3 |

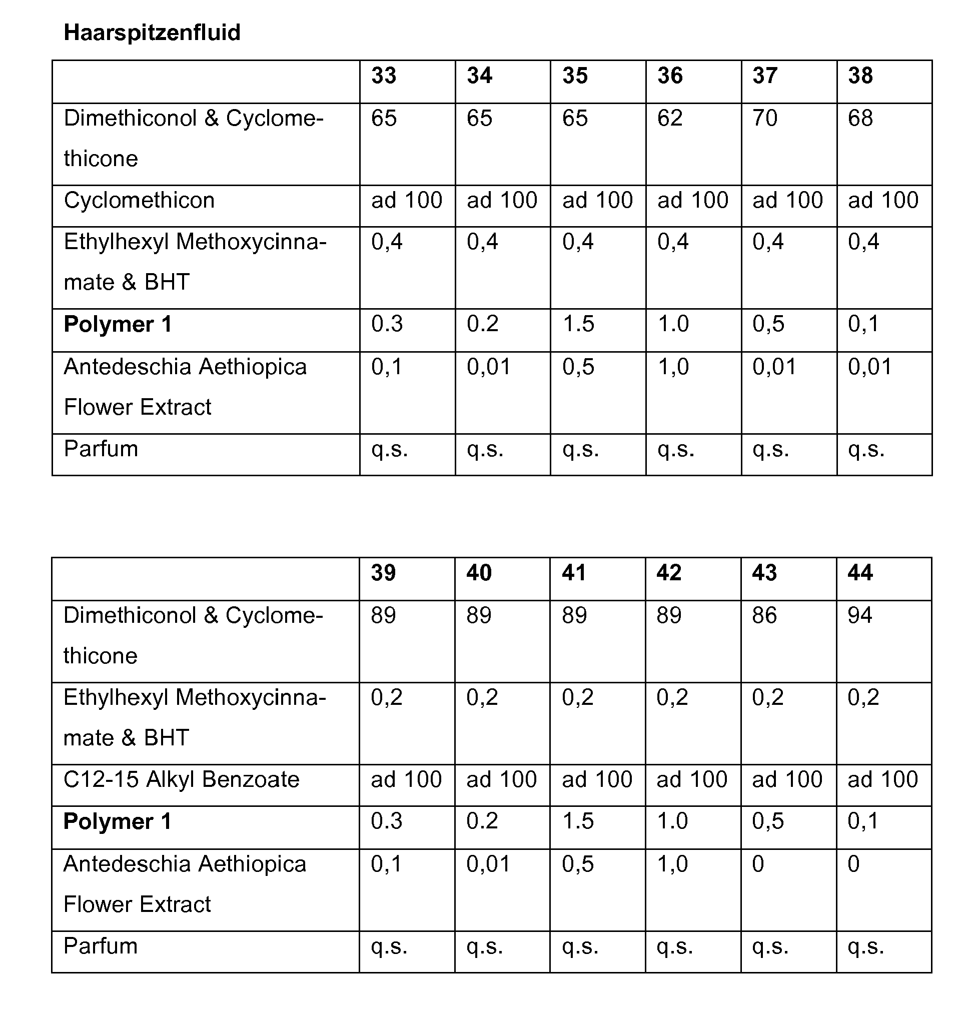

**Haarkuren**

| | **45** | **46** | **47** | **48** | **49** | **50** | **51** |
|---|---|---|---|---|---|---|---|
| Hydroxypropylmethylcellulose | 0,5 | 0,5 | 0,5 | 0,3 | 0,4 | 0,5 | 0,25 |
| Cetrimoniumbromid | 1,0 | - | 0,8 | - | 0,5 | 0,7 | 1,0 |
| Behentrimoniumchlorid | - | 0,7 | 0,3 | - | - | - | 0,1 |
| Distearoylethyl Hydroxyethylmonium Methosulfate | - | - | - | 1,2 | - | 0,7 | - |
| Palmitamidopropyltrimonium Chlorid | - | - | - | - | 0,5 | - | - |
| Glycerin | 3,5 | 8,5 | 3,0 | 2,5 | 2,0 | - | 8,5 |
| Cetearylalkohol | 2,5 | 2,0 | 3,5 | 2,5 | 2,0 | 3,5 | 4,8 |
| Glycerylstearat | 2,0 | 2,0 | 2,0 | 2,0 | 2,0 | 1,8 | 2,0 |
| Polyquaternium-10 | 0,1 | - | - | - | 0,1 | - | 0,1 |
| Guar Hydroxypropyl Trimonium Chlorid | - | 0,2 | - | 0,1 | - | - | - |
| Bis-Diglyceryl Polyacyladipate-2 | 2,0 | 2,0 | 2,0 | 2,0 | 2,0 | 2,0 | 2,0 |
| **Polymer 1** | 0,5 | 0,5 | 1,0 | 1,0 | 0,5 | 0,5 | 0,5 |
| Antedeschia Aethiopica Flower Extract | 0,1 | 0,1 | 0,1 | 0,01 | 0,1 | 0,1 | 0,1 |
| Panthenol | 0,1 | - | 0,1 | 0,1 | - | 0,15 | 0,15 |
| Oryzanol | 0,05 | 0,05 | 0,05 | - | 0,05 | 0,05 | 0,05 |
| Beads (Lactose, Cellulose, Cl 77007, Hydroxypropyl Methylcellulose) | 0,2 | - | 0,2 | 0,2 | 0,2 | 0,2 | 0,2 |
| Konservierungsmittel, Parfüm, pH-Einstellung und Lösungsvermittler | q.s. | q.s. | q.s. | q.s. | q.s. | q.s. | q.s. |
| Wasser | ad 100 | ad 100 | ad 100 | ad 100 | ad 100 | ad 100 | ad 100 |

**Leave on- Haarkuren**

| | **52** | **53** | **54** | **55** | **56** | **57** | **58** |
|---|---|---|---|---|---|---|---|
| Cetylalcohol | 1,5 | 1,8 | 2,0 | - | 1,0 | 1,7 | 2,0 |
| Cetrimoniumchlorid | 0,3 | 0,1 | 0,5 | 0,5 | - | 0,2 | - |
| Behentrimoniumchlorid | - | 0,2 | - | - | - | - | 0,2 |
| Distearoylethyl Hydroxyethylmonium Methosulfate | - | 2 | - | - | 0,5 | - | 0,2 |
| Palmitamidopropyltrimonium Chlorid | - | - | - | - | - | 0,2 | - |
| Benzophenone-4 | 0,05 | 0,03 | - | 0,1 | - | 0,1 | - |
| PVP/VA Copolymer (Luviskol VA 64 W) | 0,4 | - | - | - | 0,3 | - | - |
| Polyquaternium-37 | - | - | - | 1,0 | - | - | - |
| Polyquaternium-4 | - | 0,1 | - | 0,2 | - | 0,1 | - |
| Polyquaternium-10 | - | - | 0,5 | - | 0,3 | - | 0,2 |
| Glycerin | 8.5 | - | 10 | 0,5 | - | - | 5 |
| Amodimethicone + Trideceth-12 + Cetrimmoniumchloride | 1 | 2 | 1,5 | - | 0,5 | - | - |
| Cyclomethicone | 0.5 | 1 | - | 2 | - | - | - |
| Ceteareth-20 | 0,5 | 1 | - | - | 0,25 | - | - |
| Dimethicone | 0,5 | 2 | - | - | - | 1 | - |
| Hydroxyethylcellulose (Tylose H4000) | - | - | - | 0,3 | - | - | 0,2 |
| Hydroxypropyl Methylcellulose | 0,2 | - | 0,3 | - | 0,5 | - | 1 |
| Acrylates/C10-30 Alkyl Acrylates Crosspolymer (Carbopol ETD 2020) | 0,5 | 0,3 | 0,2 | - | - | - | - |
| C12-13 Alkyl Lactate | 2,0 | 1,0 | 1,5 | 1,0 | 1,5 | 1,0 | 1,0 |
| Laureth-4 | - | - | - | 0,5 | - | - | - |
| Aluminium Starch Octenylsuccinate | - | - | - | 1,0 | - | - | - |
| Dicaprylyl Carbonate | - | - | - | 1,0 | - | - | - |
| **Polymer 1** | 0,5 | 0,3 | 0,5 | 0,5 | 0,3 | 0,5 | 0,5 |
| Panthenol | 0,1 | - | 0,1 | 0,15 | - | 0,5 | 0,1 |
| Oryzanol | 0,05 | 0,05 | - | 0,05 | 0,05 | 0,05 | - |
| Konservierungsmittel, Parfüm, pH-Einstellung und Lösungsvermittler | q.s. | q.s. | q.s. | q.s. | q.s | q.s | q.s |
| Wasser | ad 100 | ad 100 | ad 100 | ad 100 | ad 100 | ad 100 | ad 100 |

Die Evaluierung der Formulierungen im Halbseitentest an mindestens 10 Probanden von jeweils mindestens zwei geschulten Personen zeigt sich für alle Beispiele eine deutlich verbesserte Styling-Wirkung (insbesondere verbessertes Volumen) im direkten Vergleich mit Formeln ohne Polymer 1.

## Patentansprüche

1. Haarkosmetisches Mittel, enthaltend ein kationisch amphoteres Polymer, enthaltend die Monomere Vinylpyrrolidon, *tert*-Butylacrylat, Methacrylsäure, Dimethylaminopropylmethacrylamid und Methacryloyloxyethyldimethylethyl Ammonium Ethylsulfat, mit den Vorgaben, dass der Anteil Methacrylsäure geringer ist als der Anteil Dimethylaminopropylmethacrylamid und dass das Polymer mit einer geeigneten Säure neutralisiert ist und mindestens ein weiterer Stoff der den Zustand des Haares nach Applikation verbessert.

2. Haarkosmetisches Mittel nach Anspruch 1, **dadurch gekennzeichnet, dass** das kationisch amphotere Polymer zusammengesetzt ist aus 45 bis 49 Gew.-% *tert*-Butylacrylat, 23 bis 27 Gew.-% Vinylpyrrolidon, 9 bis 11 Gew.-% Dimethylaminopropylmethacrylamid, 13 bis 17 Gew.-% Methacryloyloxyethyldimethylethyl Ammonium Ethylsulfat, 2 bis 4 Gew.-% Methacrylsäure.

3. Haarkosmetisches Mittel nach einem der vorangehenden Patentansprüche **dadurch gekennzeichnet, dass** das kationisch amphotere Polymer zusammengesetzt ist aus 47 Gew.-% tert. Butylacrylat, 25 Gew.-% Vinylpyrrolidon, 10 Gew.-% Dimethylaminopropylmethacrylamid, 15 Gew.-% Methacryloyloxyethyldimethylethyl Ammonium Ethylsulfat, 3 Gew.-% Methacrylsäure.

4. Haarkosmetisches Mittel nach einem der vorangehenden Patentansprüche **dadurch gekennzeichnet, dass** das kationisch amphotere Polymer ein Molekulargewicht von 80 000 bis 240 000 g/mol aufweist.

5. Haarkosmetisches Mittel nach einem der vorangehenden Patentansprüche **dadurch gekennzeichnet, dass** es zusätzlich 2-25% eines Tensids, ausgewählt unter den nichtionischen, anionischen, kationischen oder amphoteren Tensiden enthält.

6. Verwendung eines haarkosmetischen Mittels nach den vorangegangenen Ansprüchen zur Verbesserung des Frisurvolumens nach Ausspülen des haarkosmetischen Mittels.

7. Haarkosmetisches Mittel zur Reinigung des Haares nach einem der vorangehenden Patentansprüche enthaltend anionische Teside.

8. Haarkosmetisches Mittel zur Reinigung des Haares nach einem der vorangehenden Patentansprüche enthaltend anionische Teside von 4 bis 15 Gew.-%.

9. Haarkosmetisches Mittel zur Reinigung des Haares nach einem der vorangehenden Patentansprüche enthaltend zusätzlich amphotere Tenside besonders bevorzugt Acyl-/dialkylethylendiamine, insbesondere Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat.

10. Haarkosmetisches Mittel zur Reinigung des Haares nach einem der vorangehenden Patentansprüche enthaltend als anionische Teside Alkylethersulfate, insbesondere Laurylethersulfat in Kombination mit Cocamidopropylbetain, besonders bevorzugt die Kombination Alkylethersulfate, Cocamidopropylbetain und Alkylpolyglykolestersulfosuccinate, ganz besonders bevorzugt Laurylethersulfat, Cocamidopropylbetain und Alkylpolyglykolestersulfosuccinate, einzusetzen.

11. Haarkosmetisches Mittel zur Pflege des Haares nach einem der Patentansprüche 1 bis 6 enthaltend kationische Teside.

12. Haarkosmetisches Mittel zur Pflege des Haares nach einem der Patentansprüche 1 bis 6 und 11 enthaltend kationische Teside von 0,1 bis 2 Gew.-%.

13. Haarkosmetisches Mittel zur Pflege des Haares nach einem der Patentansprüche 1 bis 6 und 11 bis 12 enthaltend zusätzlich Filmbildner.

14. Haarkosmetisches Mittel zur Pflege des Haares nach einem der Patentansprüche 1 bis 6 und 11 bis 13 enthaltend als kationische Teside Alkylamine, Alkylimidazole, Ethoxylierte Amine, Quaternäre Tenside, Esterquats.

15. Haarkosmetisches Mittel zur Pflege des Haares nach einem der Patentansprüche 1 bis 6 und 11 bis 14 enthaltend als Filmbildner Cellulosederivate, quaternisierte Guar Gum Derivate und Dimethyldiallylammoniumchlorid-Acrylamid Copolymere.
